Europäisches Patentamt

European Patent Office

Office européen des brevets

(19)

(11) Veröffentlichungsnummer: **0 139 040**
**A2**

(12) # EUROPÄISCHE PATENTANMELDUNG

(21) Anmeldenummer: **83111396.4**

(22) Anmeldetag: **15.11.83**

(51) Int. Cl.⁴: **A 61 M 5/14**

(30) Priorität: **25.10.83 DE 8330608 U**
**25.10.83 DE 8330609 U**

(43) Veröffentlichungstag der Anmeldung:
**02.05.85 Patentblatt 85/18**

(84) Benannte Vertragsstaaten:
**AT BE CH DE FR GB IT LI LU NL SE**

(71) Anmelder: **pfm Plastik für die Medizin**
**Oberbuschweg 76 Postfach 50 17 48**
**D-5000 Koln 50 (Sürth)(DE)**

(72) Erfinder: **Fischer, Bernd Michael**
**In der Kemperhecke 9**
**D-5204 Lohmar 1(DE)**

(74) Vertreter: **Hanewinkel, Lorenz, Dipl.-Phys. et al,**
**Patentanwälte Dr.Ing. Heinz Nickels Dipl.-Phys. Lorenz**
**Hanewinkel Detmolder Strasse 26**
**D-4800 Bielefeld 1(DE)**

(54) **Infusionseinrichtung.**

(57) Die Infusionseinrichtung weist einen Zuführschlauch (3) auf, der an seinem abströmseitigen Längenende mit einem V- oder Y-förmigen Verbindungsstück (6) verbindbar und mit seinem zuströmseitigen Längenende an eine Infusionszuführung anschließbar bzw. angeschlossen ist. Dabei ist der Zuführschlauch (3) mit seinem abströmseitigen Längenende an einen seitlichen, spitzwinklig zur Strömungsrichtung (A) in ein geradliniges Rohrstück (4) einmündenden Rohrstutzen (5) des einteiligen Verbindungsstückes (6) fest angeschlossen und bildet mit dem Verbindungsstück (6) ein Einsatzteil.

Diese Infusionseinrichtung ist einerseits als Infusionsverbindungsleitung und andererseits als Infusionsgerät einsetzbar, wobei eine Zusatzinjektion direkt im Anschlußbereich des Katheters (7) erfolgt.

Weiterhin läßt sich diese Infusionseinrichtung mit gleichen Einrichtungen übereinander stecken und bildet dann ein Mehrfach-Infusionsgerät.

Fig. 3

EP 0 139 040 A2

" Infusionseinrichtung "

Die Erfindung bezieht sich auf eine Infusionseinrichtung in Form einer Infusionsverbindungsleitung und eines Infusionsgerätes, welche einen Schlauch und ein an einem Schlauch-Längenende angesetztes, ein geradlinig verlaufendes Rohrstück und einen in Strömungsrichtung der Infusionslösung spitzwinklig darin einmündenden, seitlichen Rohrstutzen zeigendes, einteiliges Verbindungsstück aufweist.

Bei bekannten Infusionsverbindungsleitungen ist der Schlauch an das abströmseitige Ende des geradlinigen Rohrstückes des Verbindungsstückes angeschlossen und der seitlich schräg abgehende Rohrstutzen ermöglicht eine Zusatzinjektion während der Behandlung. Hierbei ist es nachteilig, daß bei der Zusatzinjektion das Injektionsmittel erst die gesamte Länge des Schlauches durchfließen muß, bis es in den Katheter bzw. an die Behandlungsstelle herankommt; dadurch tritt einerseits eine Zeitverzögerung in der Zusatzinjektion auf und desweiteren lassen sich durch den langen Strömweg Verluste an Injektionsmittel nicht vermeiden, was insbesondere bei kleinen Injektionsmengen äußerst nachteilig ist.

0139040

Bei bekannten Infusionsgeräten hat der Schlauch ein von einem Luer-Lok-Ansatz mit Überwurfkappe gebildetes Verbindungsglied und wird mit diesem Verbindungsglied an ein V- bzw. Y-artiges Verbindungsstück angeschlossen, an dem ein Katheter sitzt.

Sollten nunmehr mehrere Infusionsmittel gleichzeitig dem Patienten zugeführt werden, dann werden mehrere Infusionsgeräte eingesetzt und dabei jedes Gerät mit seinem Schlauch an ein Mehrfach-Verbindungsstück angeschlossen.

Nachteilig bei den bekannten Infusionsgeräten ist einmal die manuelle Verbindung von Schlauch und Verbindungsstück und zum anderen die Erfordernis unterschiedlicher Verbindungsstücke, nämlich mit zwei, drei oder mehreren Anschlüssen für die Schläuche.

Aufgabe der Erfindung ist es deshalb, unter Vermeidung der bisherigen Mängel eine Infusionseinrichtung mit günstiger Anordnung des Schlauches am Verbindungsstück zu schaffen, wodurch eine gezielte Zusatzinjektionsmöglichkeit gewährleistet wird und gleichzeitig mehrere Infusionseinrichtungen in einfacher und sicherer Weise zu einer Zwei- oder Mehrfacheinrichtung zusammensetzbar sind.

Diese Aufgabe wird erfindungsgemäß dadurch gelöst, daß der Schlauch mit seinem abströmseitigen Längenende an dem seitlichen, schrägen Rohrstutzen des Verbindungsstückes fest angeschlossen ist.

Das zuströmseitige Ende des geradlinigen Rohrstückes des Verbindungsstückes dient zur Zusatzinjektion und trägt einen lösbaren Verschluß bzw. Injektionsstopfen.

Das abströmseitige Ende des Rohrstückes des Verbindungsstückes steht mit einem Katheter in lösbarer oder fester Verbindung.

Die beiden Längenenden des geradlinigen Rohrstückes des Verbindungsstückes sind als Verbindungsglieder ausgeführt, durch die eine Übereinanderreihung zweier oder mehrerer Einrichtungen zur Schaffung eines
Mehrfach-Infusionsgerätes möglich ist.

Der Gegenstand der Erfindung erstreckt sich nicht nur auf die Merkmale der einzelnen Ansprüche, sondern auch auf deren Kombination.

Durch den festen Anschluß des Schlauches mit seinem abströmseitigen
Ende am Verbindungsstück fließt die durch den Schlauch zugeführte
Infusionslösung über das Verbindungsstück direkt in den daran
angeschlossenen Katheter, so daß diese erfindungsgemäße Verbindung
zwischen Schlauch und Verbindungsstück eine Umkehrung zu der
bekannten Verbindungsart darstellt.

Daraus ergibt sich, daß die Zusatzinjektion direkt im Einflußbereich der
in den Katheter einfließenden Infusionslösung auch in den Katheter
eingebracht werden kann, in dem hierfür das geradlinig verlaufende
Rohrstück benutzt wird und somit die Zusatzinjektion ohne langen
Strömungsweg (Zeitverlust) und ohne Volumenverlust gezielt an die
Behandlungsstelle herangebracht werden kann; es ist somit ein direktes
Zuspritzen des Injektionsmittels möglich geworden.

Weiterhin stellt die feste Verbindung zwischen Schlauch und Verbindungsstück eine verbesserte hygienische Ausführung dar, da zusätzliche Verbindungsmaßnahmen ausgeschlossen werden.

Bei der Ausführung der Infusionseinrichtung als Infusionsverbindungsleitung läßt sich diese einwandfrei hygienisch verpacken und äußerst
leicht für den Gebrauch einsetzen sowie kann mit gleichen Leitungen am
Zusatzinjektionsansatz mit einem Handgriff verbunden werden, so daß
diese Leitung einen hohen Gebrauchswert bei vielseitigem Einsatz hat.

Bei der Ausführung der erfindungsgemäßen Infusionseinrichtung als Infusionsgerät ist der von der Tropfkammer abgehende Schlauch fest (unlösbar) mit einem Y- bzw. V-Verbindungsstück verbunden, so daß hierdurch neben der hygienischen Verbindungsausführung der bisher übliche Luer-Lok-Verschluß entfällt, was sich kosten- und handhabungsmäßig auf das gesamte Gerät auswirkt.

Die Tropfkammer mit Schlauch und Verbindungsstück stellt eine herstellungsmäßige und leicht verpackbare hygienische Einheit dar, wobei an das Verbindungsstück bei der Benutzung ein Katheter anschließbar ist oder aber das Verbindungsstück ebenfalls herstellungsseitig bereits mit einem Katheter ausgestattet ist. Trotz der festen Verbindung zwischen Schlauch und Verbindungsstück ist durch das Verbindungsstück hindurch eine Zusatzinjektion in den Katheter möglich.

Ein weiterer großer Vorteil der einteiligen Infusionseinrichtung wird in der einfachen, sicheren und leicht durchführbaren Verbindungsmöglichkeit von zwei oder mehreren Infusionseinrichtungen zu einem Mehrfach-Infusionsgerät gesehen, in dem die Verbindungsstücke durch Steckverbindung und Überwurfkappen übereinandersetzbar sind - hierbei laufen alle Schläuche seitlich in die Verbindungsstücke ein und diese sind geradlinig übereinanderverlaufend miteinander verbunden. Es ist somit während der Behandlung möglich, zusätzliche Infusionseinrichtungen anzuschließen, indem vom oberen Infusionsgerät der Verschluß- bzw. Injektionsstopfen abgenommen und dann ein weiteres Gerät aufgesteckt werden kann.

Da das Infusionsgerät bereits mit einem Verbindungsstück ausgestattet ist, entfallen einerseits die bisherigen Luer-Lok-Verbindungen zwischen Schlauch und Verbindungsstück und zum anderen die jeweils einzusetzenden Mehrfach-Verbindungsstücke, sondern diese sind durch die zusammensteckbaren Verbindungsstücke des Schlauches bildbar. Dieses Infusionsgerät hat trotz seiner einfachen Ausführung eine wesentliche verbesserte Handhabung und Behandlungsmöglichkeit geschaffen und dadurch einen hohen Gebrauchswert erhalten.

Anhand der Zeichnungen werden nachfolgend Ausführungsbeispiele gemäß der Erfindung näher erläutert. Es zeigt:

Fig. 1 eine Seitenansicht einer aus einem Schlauch und einem Verbindungsstück gebildeten Infusionsverbindungsleitung mit lösbarer Anschlußmöglichkeit eines Katheters an dem Verbindungsstück,

Fig. 2 eine Seitenansicht derselben Infusionsverbindungsleitung, jedoch mit fest an das Verbindungsstück angeschlossenen Katheter,

Fig. 3 eine Seitenansicht eines Infusionsgerätes mit Tropfkammer, Schlauch und Verbindungsstück sowie lösbar am Verbindungsstück festgelegtem Katheter,

Fig. 4 eine Seitenansicht desselben Infusionsgerätes, jedoch mit fest an das Verbindungsstück angesetztem Katheter und in den Schlauch eingesetzter Pumpe,

Fig. 5 eine Seitenansicht mehrerer durch die Verbindungsstücke zusammensetzbar bzw. zusammengesetzer Infusionsgeräte,

Fig. 6 eine schematische Darstellung eines Mehrfach-Infusionsgerätes mit katheterseitigem Zuspritz-Verbindungsstück.

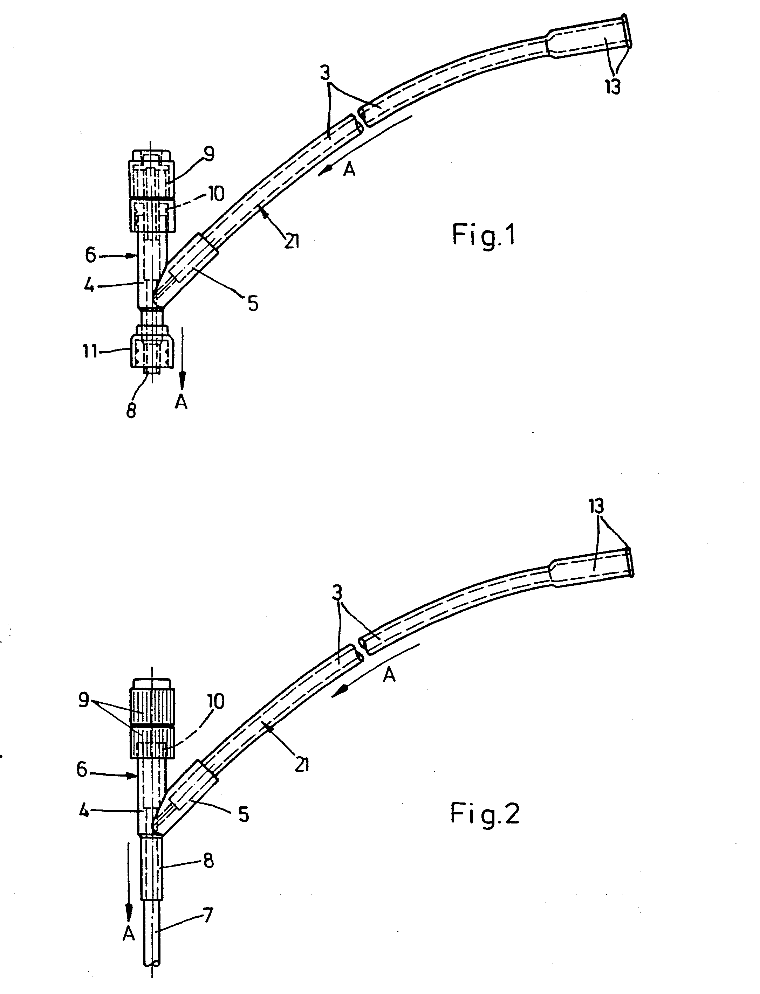

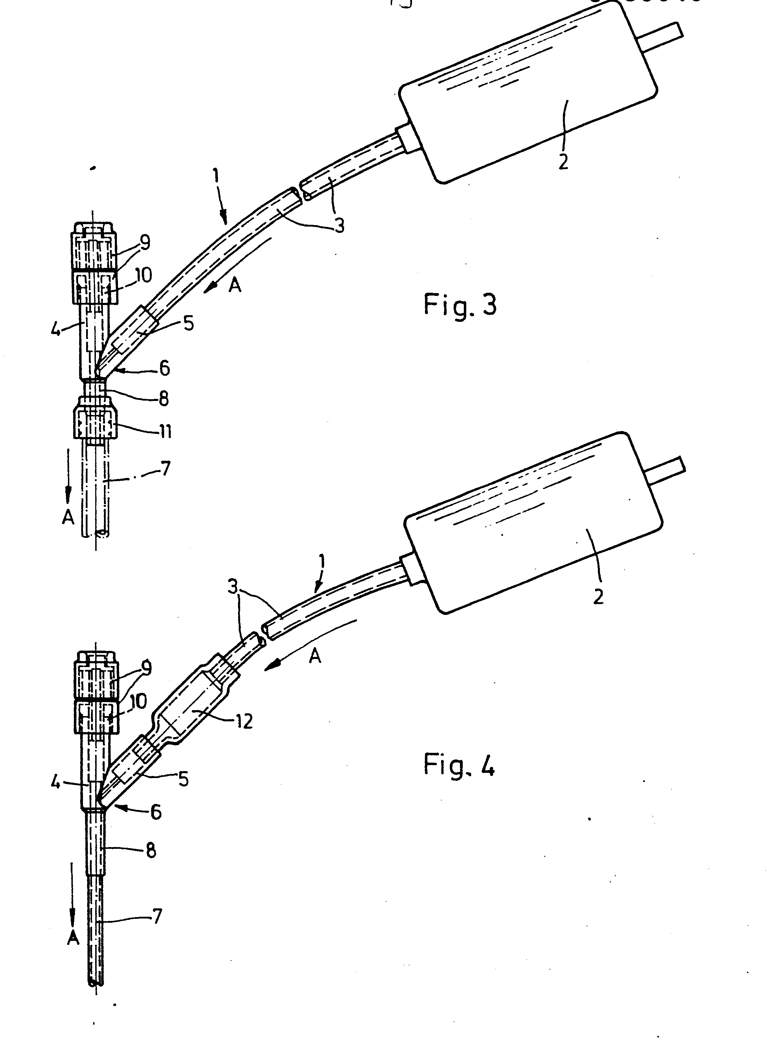

Die erfindungsgemäße Infusionseinrichtung ist als Infusionsverbindungsleitung (Fig. 1 und 2 ) und als Infusionsgerät (Fig. 3 bis 6) ausgebildet, wobei beide Teile einzeln oder zusammen eingesetzt werden können; beide Ausführungen haben dieselben erfindungsgemäßen Grundsatzmerkmale und dienen der Infusionszuführung und Zusatzinjektion.

Zunächst wird auf die Infusionseinrichtung gemäß Fig. 1 und 2 Bezug genommen, die eine Infusionsverbindungsleitung 1 darstellt.

Mit 3 ist ein Zuführschlauch aus vorzugsweise durchsichtigem Kunststoff und mit 6 ein einteiliges Verbindungsstück aus vorzugsweise durchsichtigem oder farbigem Kunststoff der Infusionsverbindungsleitung 1 bezeichnet.

Das Verbindungsstück 6 weist ein geradlinig verlaufendes Rohrstück 4 und einen in Strömungsrichtung "A" spitzwinklig darin einmündenden, seitlichen Rohrstutzen 5 auf. Dieses Verbindungsstück 6 stellt einen sogenannten V- oder Y-Verbinder dar.

An den schrägstehenden Rohrstutzen 5 des Verbindungsstückes 6 ist der Schlauch 3 mit seinem abströmseitigen Längenende fest angeschlossen, vorzugsweise durch Schweißen befestigt.

Das andere, zuströmseitige Längenende des Schlauches 3 ist mit einem Verbindungsansatz 13, vorzugsweise einem Luer-Lok-Ansatz, ausgestattet.

Das geradlinige Rohrstück 4 zeigt an seinem zuströmseitigen Ende einen Verbindungsansatz 1o, vorzugsweise einen Luer-Lok-Ansatz, und auf diesem Verbindungsansatz 1o ist ein Verschluß- bzw. ein durchstechbarer Injektionsstopfens lösbar festgelegt, so daß durch diesen Stopfen 9 hindurch in das Rohrstück 4 eine Zuspritzung (Zusatzinjektion) während des Infusionsflusses (der Behandlung) möglich ist.

Das Rohrstück 4 ist an seinem abströmseitigen Ende zu einem Verbindungsglied 8, vorzugsweise einem Luer-Lok-Ansatz, geformt, auf dem eine bewegliche (drehbare) Überwurfkappe 11 (Fig. 1) angeordnet ist. An diesem Verbindungsglied 8 läßt sich durch die Überwurfkappe 11 ein Katheter lösbar befestigen. Es liegt im Rahmen der Erfindung, das Verbindungsglied 8 auch mit starrer Überwurfkappe 11 auszustatten.

Gemäß der weiteren Ausführung nach Fig. 2 ist an dem ansatzförmigen Verbindungsglied 8 ein Katheter 7 fest angeschlossen, vorzugsweise durch Schweißen, befestigt.

Das Infusionsgerät nach Fig. 3 bis 6 ist insgesamt mit der Bezugszahl 21 bezeichnet und weist eine Tropfkammer 2 auf, an die ein Zuführschlauch 3 mit einem Längenende angeschlossen ist.

Das andere Längenende des Schlauches 3, und zwar das abströmseitige Schlauch-Längenende, ist mit einem seitlichen, spitzwinklig zur Strömrichtung "A" in ein geradliniges Rohrstück 4 einmündenden Rohrstutzen 5 eines einteiligen Verbindungsstückes 6 fest verbunden, so daß die Tropfkammer 2, der Schlauch 3 und das einen Y- bzw. V-Verbinder darstellende Verbindungsstück 6 eine Einheit bilden.

Der Infusionsflüssigkeitszulauf erfolgt somit aus dem Schlauch 3 heraus seitlich in das Verbindungsstück 6 hinein und von dort in einen an das abströmseitige Ende des geradlinigen Rohrstückes 4 fest oder lösbar angeschlossenen Katheter 7. Das abströmseitige Ende des Rohrstückes 4 ist für den Katheteranschluß mit einem Verbindungsglied 8 in Form eines Ansatzes ausgestattet.

Das Rohrstück 4 des Verbindungsstückes 6 trägt am zuströmseitigen Ende einen lösbaren Verschluß- bzw. Injektionsstopfen 9, wobei der durchsteckbare Injektionsstopfen 9 eine Zusatzinjektion ermöglicht und der abnehmbare Verschluß- oder Injektionsstopfen 9 nach der Abnahme eine Verbindung zweier Verbindungsstücke 6 und somit zweier Infusionsgeräte 1 gestattet.

Das zuströmseitige Ende des Rohrstückes 4 ist zur lösbaren Befestigung des Stopfens 9 sowie für die Verbindung benachbarter Verbindungsstücke 6 mit einem Verbindungsansatz lo, vorzugsweise einem Luer-Lok-Ansatz, ausgestattet, auf dem der Stopfen 9 lösbar festgelegt ist.

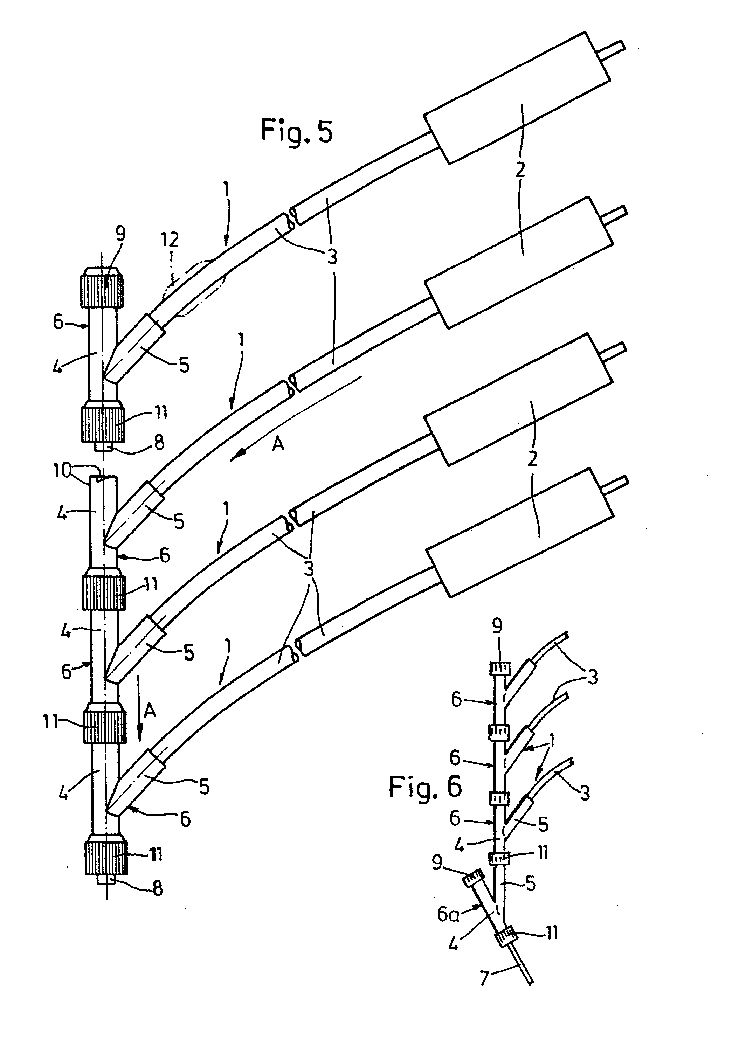

Das abströmseitige Ende des Rohrstückes 4 ist zu dem Verbindungsglied 8 geformt, welches ebenfalls als Luer-Lok-Ansatz ausgebildet ist und um den eine vorzugsweise bewegliche Überwurfkappe 11 angeordnet ist, die bei einem Einzel-Infusionsgerät 21 (Fig. 3) für die lösbare Festlegung des Katheters 7 dient und desanderen für die Verbindung mehrerer Infusionsgeräte 21 gemäß Fig. 5 im Bereich der Verbindungsstücke 6 genutzt wird.

Gemäß Ausführung nach Fig. 4 ist das Verbindungsglied 8 als Ansatz (Rohrstück) ausgebildet, an dem der Katheter 7 fest, vorzugsweise durch Schweißen, angebracht ist.

Wie Fig. 4 weiter zeigt, läßt sich in den Schlauch 3 eine balgartige Pumpe 12 einsetzen, um Infusionsflüssigkeit nachpumpen zu können. Beispielsweise ist die aus elastischem Material, wie Gummi oder Kunststoff, bestehende, rohr- bzw. schlauchförmige, jedoch zur Pumpwirkung auf einem Längenbereich erweiterte Pumpe 12 mit einem Ende am Rohrstutzen 5 befestigt und nimmt am anderen Ende das Schlauchende des Schlauches 3 auf.

Die behälterförmige Tropfkammer 2, der Schlauch 3 und das einteilige Verbindungsstück 6 sind in bevorzugter Weise aus durchsichtigem oder farbigem Kunststoff hergestellt.

Das einzelne Infusionsgerät 21 besteht aus der Kammer 2, dem Schlauch 3 und dem V- oder Y-förmigen einteiligen Verbindungsstück 6 mit Verschluß- bzw. Injektionsstopfen 9 und dem Anschluß 8,11 für die lösbare Verbindung mit dem Katheter 7 oder dem Ansatz 8 für die feste Katheterverbindung. Diese Einheit 21 ermöglicht eine Infusionszuführung und eine zusätzliche Injektion während der Infusionszuführung.

Weiterhin läßt sich diese Einheit 21 mit gleichen Geräten 21 zu einer Mehrfacheinheit gemäß Fig. 5 verbinden, indem jeweils ein Verbindungsstück 6 mit seinem Verbindungsglied 8,11 auf den Verbindungsansatz 1o aufgesteckt und durch die Überwurfkappe 11 verbunden wird, so daß mehrere übereinander angeordnete und miteinander verbundene Infusionsgeräte 21 zusammengefaßt sind und dadurch gleichzeitig mehrere Infusionslösungen dem Patienten über einen Katheter 7 zugeführt werden können und dabei zusätzlich noch eine Zusatzinjektion durch den Stopfen 9 des obersten Infusionsgerätes 21 möglich ist.

In Fig. 5 ist eine aus mehreren Infusionsgeräten 21 durch die Luer-Lok-Verbindung 8,11,1o zusammengesetzte Baueinheit dargestellt, wobei das in der Zeichnung oberste Gerät 21 noch nicht mit dieser Einheit verbunden ist, sondern von dieser Einheit der Stopfen 9 des oberen Verbindungsstückes 6 abgenommen worden ist, so daß das darüber mit Abstand gezeichnete Gerät 21 durch die Luer-Lok-Verbindung 8,1o,11 zusammengesteckt und verbunden werden kann, und zwar lösbar.

Das unterste Infusionsgerät 21 wird entweder von einem Gerät 21 mit fest angeschweißtem oder durch die Luer-Lok-Verbindung 8,11 lösbaren Katheter 7 gebildet.

Um bei einem Mehrfach-Infusionsgerät einen langen Zuspritzweg für die Zusatzinjektion zum Katheter 7 zu vermeiden, ist gemäß Fig. 6 an das unterste Injektionsgerät 21 ein Verbindungsstück 6a mit seinem Rohrstutzen 5 an das Verbindungsstück 6 angesetzt und durch die Luer-Lok-Verbindung 8,9 lösbar gehalten. Hierbei erstreckt sich der Rohrstutzen 5 des Verbindungsstückes 6a in geradliniger Verbindung zum Rohrstück 4 des darüber befindlichen Verbindungsstückes 6 und sein geradliniges Rohrstück 4 schräg dazu, so daß durch den Injektionsstopfen 9 eine Zuspritzmöglichkeit nahe dem Katheter 7 durch dieses Verbindungsstück 6a erfolgen kann.

An das abströmseitige Ende des Rohrstückes 4 des Verbindungsstückes 6a ist ein Katheter 7 durch die Luer-Lok-Verbindung 8,11 lösbar oder, an den Ansatz 8 angeschweißt, fest verbunden.

Patentansprüche

1. Infusionseinrichtung, die einen Zuführschlauch aufweist, der an seinem abströmseitigen Längenende mit einem V- oder Y-förmigen Verbindungsstück verbindbar und mit seinem zuströmseitigen Längenende an eine Infusionszuführung anschließbar bzw. angeschlossen ist, dadurch gekennzeichnet, daß der Zuführschlauch (3) mit seinem abströmseitigen Längenende an einen seitlichen, spitzwinklig zur Strömungsrichtung (A) in ein geradliniges Rohrstück (4) einmündenden Rohrstutzen (5) des einteiligen Verbindungsstückes (6) fest angeschlossen ist und mit dem Verbindungsstück (6) ein Einsatzteil bildet.

2. Infusionseinrichtung nach Anspruch 1, dadurch gekennzeichnet, daß der Schlauch (3) durch Schweißen an dem Rohrstutzen (5) des Verbindungsstückes (6) befestigt ist.

3. Infusionseinrichtung nach Anspruch 1, dadurch gekennzeichnet, daß das Rohrstück (4) des Verbindungsstückes (6) an seinem zuströmseitigen Ende einen Verbindungsansatz (lo) aufweist und darauf ein eine Zusatzinjektion ermöglichender, durchstechbarer Verschlußstopfen (9) lösbar festgelegt ist.

4. Infusionseinrichtung nach den Ansprüchen 1 bis 3, dadurch gekennzeichnet, daß das Rohrstück (4) des Verbindungsstückes (6) an seinem abströmseitigen Ende zu einem ansatzförmigen Verbindungsglied (8) ausgebildet ist, auf dem eine vorzugsweise bewegliche Überwurfkappe (11) für den lösbaren Anschluß eines Katheters (7) angeordnet ist.

5.   Infusionseinrichtung nach den Ansprüchen 1 bis 3, dadurch gekennzeichnet, d a ß das Rohrstück (4) des Verbindungsstückes (6) am abströmseitigen Ende zu einem ansatzförmigen Verbindungsglied (8) ausgebildet ist, an dem ein Katheter (7) fest angeschlossen, vorzugsweise durch Schweißen befestigt ist.

6.   Infusionseinrichtung nach Anspruch 1 und 2, dadurch gekennzeichnet, d a ß der Schlauch (3) an seinem zuströmseitigen Längenende einen Verbindungsansatz (13) aufweist.

7.   Infusionseinrichtung nach den Ansprüchen 1 bis 6, dadurch gekennzeichnet, d a ß die Verbindungssätze (1o,8/13) des Verbindungsstückes (6) und des Schlauches (3) in bekannter Weise von Lüer-Lok-Ansätzen gebildet sind.

8.   Infusionseinrichtung, insbesondere nach den Ansprüchen 1 bis 7, dadurch gekennzeichnet, d a ß der Schlauch (3) mit seinem zuströmseitigen Längenende an eine Tropfkammer (2) und mit seinem abströmseitigen Längenende an den seitlichen Rohrstutzen (5) des einteiligen Verbindungsstückes (6) fest angeschlossen ist und mit der Tropfkammer (2) und dem Verbindungsstück (6) ein Infusionsgerät (1) bildet, wobei das Verbindungsstück (6) am zuströmseitigen Ende des Rohrstückes (4) einen lösbaren Verschluß- bzw. Injektionsstopfen (9) trägt sowie am abströmseitigen Ende ein Verbindungsglied (8) für einen Katheteranschluß und eine Verbindungsgliedaneinanderreihung aufweist (Fig. 3 ).

9.   Infusionseinrichtung nach Anspruch 8, dadurch gekennzeichnet, d a ß in den Schlauch (3) eine balgartige Pumpe (12) eingesetzt ist, die vorzugsweise mit einem Ende an den Rohrstutzen (5) des Verbindungsstückes (6) fest angeschlossen ist und am anderen Ende den Schlauch (3) fest angesetzt aufnimmt.

10. Infusionseinrichtung nach Anspruch 8, dadurch gekennzeichnet, **d a ß** mehrere übereinander angeordnete, mit den Verbindungsansätzen (8,1o) der Verbindungsstücke (6) ineinandergesteckte und durch die Überwurfkappen (11) in der lösbaren Steckverbindung gesicherte Infusionsgeräte (1) ein Mehrfach- Infusionsgerät bilden (Fig. 5).

11. Infusionseinrichtung nach Anspruch 1o, dadurch gekennzeichnet, **d a ß** an das Verbindungsstück (6) des untersten Infusionsgerätes (1) eines Mehrfach-Infusionsgerätes ein Verbindungsstück (6a) mit seinem Rohrstutzen (5) durch Luer-Lok-Verbindung (8,11) lösbar angesetzt ist und sein geradliniges Rohrstück (4) steht schräg zum Rohrstück (4) des darüber angeordneten Verbindungsstückes (6), und bildet durch seinen Injektionsstopfen (9) eine seitliche Zusatzinjektion in den an das abströmseitige Ende des Rohrstückes (4) lösbar oder fest angeschlossenen Katheter (7) (Fig. 6).

Fig.1

Fig.2

0139040

Fig. 3

Fig. 4

Fig. 5

Fig. 6